# EUROPÄISCHE PATENTSCHRIFT

(11) **EP 0 973 573 B1**
(45) Veröffentlichungstag und Bekanntmachung des Hinweises auf die Patenterteilung: **01.03.2006**
(21) Anmeldenummer: 98921414.3
(22) Anmeldetag: 02.04.1998
(51) Int. Cl.: A61M 25/00, A61B 5/00

(54) **KATHETER ZUR MESSUNG CHEMISCHER PARAMETER, INSBESONDERE ZUM EINFÜHREN IN BIOLOGISCHES GEWEBE, FLÜSSIGKEITEN ODER DERGLEICHEN**
CATHETER FOR MEASURING CHEMICAL PARAMETERS, IN PARTICULAR FOR INTRODUCING INTO BIOLOGICAL TISSUES, LIQUIDS OR THE LIKE
CATHETER POUR MESURER DES PARAMETRES CHIMIQUES, A INTRODUIRE NOTAMMENT DANS DES TISSUS BIOLOGIQUES, LIQUIDES OU SIMILAIRES

(30) Priorität: 09.04.1997 DE 19714572
(43) Veröffentlichungstag der Anmeldung: 26.01.2000
(73) Patentinhaber: Disetronic Licensing AG, 3401 Burgdorf (CH)
(72) Erfinder: Haindl, Hans, Dr., 30974 Wennigsen (DE)
(74) Vertreter: Leine, Sigurd
(86) Internationale Anmeldenummer: PCT/EP1998/001906
(87) Internationale Veröffentlichungsnummer: WO 1998/044978

(56) Entgegenhaltungen:
- EP-A- 0 381 062
- EP-A- 0 401 179
- DE-A- 3 342 170
- FR-A- 2 655 548
- GB-A- 2 030 454
- US-A- 5 441 481

## Beschreibung

Die Erfindung betrifft einen Katheter der im Oberbegriff des Anspruchs 1 genannten Art zur Messung chemischer Parameter, insbesondere zum Einführen in biologisches Gewebe, Flüssigkeiten oder dergleichen.

Katheter der betreffenden Art sind allgemein bekannt. Sie werden als Mikrodialysekatheter bezeichnet und eingesetzt, um mit Hilfe der Mikrodialyse bestimmte chemische Parameter im Inneren des biologischen Gewebes, in der Regel des menschlichen Körpers, zu messen. Ein typisches Beispiel eines solchen chemischen Parameters ist der Gehalt des Blutzuckers im subkutanen Gewebe. Das Prinzip dieser Messung, das z. B. in DE 40 01 760 C2 und in WO 95/32746 beschrieben ist, besteht darin, daß eine doppellumige Sonde durch die Haut eingeführt wird, die an ihrer Spitze einen halbdurchlässigen Anteil hat. Eine Spüllösung wird durch einen Kanal in den Katheter in diese Sonde gepumpt, fließt an der halbdurchlässigen Membran vorbei und nimmt dabei den Blutzucker aus dem subkutanen Gewebe auf. Durch einen zweiten Kanal in dem Katheter wird diese mit Blutzucker angereicherte Spüllösung wieder aus dem Körper herausgeführt, außerhalb des Körpers mit einer Glukose-Oxidase-Lösung gemischt und einem Sensor zugeführt, der die Reaktionsprodukte der Glukose-Oxidase-Reaktion mißt. Mit einer solchen Anordnung ist mit relativ geringer Verzögerung die kontinuierliche Messung des Blutzuckers möglich.

Mikrodialysesonden zur Durchführung der zuvor beschriebenen Messung sind beispielsweise in US 5 106 365 und WO 95/20983 bekannt. Durch DE 33 42 170 A 1 ist ein Katheter bekannt, der dort als Dialysesonde bezeichnet ist und eine Dialysemembran und Leitungen für den Perfusionsströmungsmittelstrom durch die Membran umfaßt. Die Dialysemembran ist von einem Gehäuse umgeben, welches die Membran stützt und teilweise freigibt und darüber hinaus steifer als die Membran selbst ist. Die Leitungen bilden diametral in dem Katheter gegenüberliegende Kanäle, die innerhalb der schlauchförmigen Membran enden. Das verhältnismäßig steife Gehäuse soll verhindern, daß nach Einführen des Katheters in biologisches Gewebe dieses die schlauchförmige Membran zusammendrückt und dadurch deren Funktion und die Strömung innerhalb der schlauchförmigen Membran beeinträchtigt. Dieser bekannte Katheter ist durch die Anbringung des stützenden Gehäuses aufwendig und teuer in der Herstellung und erfordert außerdem einen verhältnismäßig großen Durchmesser. Ein weiterer Nachteil besteht darin, daß die Spülströmung innerhalb der schlauchförmigen Membran nicht alle Teile der Membran in gleicher Weise erfaßt.

Neben dem Nachteil der ungleichmäßigen Benetzung der teildurchlässigen Membran durch den Flüssigkeitsstrom und der dadurch bedingten erheblichen Einschränkung der Austauschfläche ist von Nachteil, daß die Sonde der betreffenden Art nicht unter Sog betrieben werden kann, weil dadurch innerhalb der schlauchförmigen Membran ein Unterdruck entstehen würde, der zu einem Zusammenfall in der Membran führen würde. Bei Betrieb unter Druck kommt ein Totraumvolumen zum Tragen, wodurch sich eine zeitliche Verzögerung des Meßergebnisses ergibt. Abgesehen davon ist bei dieser bekannten Sonde das Gesamtvolumen innerhalb der schlauchförmigen Membran verhältnismäßig groß, so daß das Verhältnis von Austauschoberfläche zu Volumen gering ist mit der Folge, daß die Konzentration in dem abfließenden Flüssigkeitsstrom gering ist und somit die Messung auf einem geringeren Niveau erfolgen muß.

Durch EP 401 174 A1 ist ein Katheter der im Oberbegriff des Anspruchs 1 genannten Art bekannt, bei der die zylindrische Wandung des äußeren Zuführkanals und des inneren Rückführkanals durch starre Rohre gebildet sind. Am Ende des äußeren starren Rohres erstreckt sich konzentrisch eine starre rohrförmige Membran, deren distales Ende über einen Kunststoffteil dicht mit dem distalen Ende des inneren rohrförmigen Teils verbunden ist. Die rohrförmige Membran ragt in einen erweiterten Teil des distalen Endes des äußeren rohrförmigen Teils und ist mit diesem durch Kunststoff verklebt. Diese Konstruktion ist aufwendig im Aufbau und erfordert wegen der Verbindung der rohrförmigen Membran mit dem äußeren rohrförmigen Teil eine komplizierte Fertigung.

Durch GB 2 030 454 ist ein Katheter ähnlich der zuvor beschriebenen bekannten Art bekannt, bei der das innere und das äußere Rohr durch Stützmittel gegeneinander abgestützt sind. In das distale Ende des inneren Rohres ist der Stiel eines pilzförmigen Pfropfens eingesetzt, dessen Pilzteil über die distalen Enden der beiden Rohre hinausragt und auf seiner halbkreisförmigen Oberfläche des Pilzteils eine Schicht aus gasdurchlässigem Material aufweist. Diese bekannte Konstruktion ist sehr kompliziert im Aufbau und erfordert mehrere Fertigungsschritte.

Der Erfindung liegt die Aufgabe zugrunde, einen Katheter der betreffenden Art zu schaffen, der die beim Stand der Technik beschriebenen Nachteile nicht aufweist, der also insbesondere einfach im Aufbau und preiswert herstellbar ist.

Die der Erfindung zugrundeliegende Aufgabe wird durch die im Kennzeichen des Anspruchs 1 angegebene Lehre gelöst.

Der Grundgedanke der Erfindung besteht darin, das äußere Teil schlauchförmig und über seine gesamte Länge aus Membranmaterial zu bilden und bis auf den vorderen Endbereich des Katheters mit einer undurchlässigen Schicht zu versehen. Das bedeutet, daß eine Verklebung zwischen dem schlauchförmigen Teil und der durchlässigen Membran entfällt und dadurch auch ein entsprechend komplizierter Arbeitsgang. Da das äußere schlauchförmige Teil über seine gesamte Länge aus Membranmaterial hergestellt wird, besteht der einzige noch durchzuführende Arbeitsgang darin, die dichtende Schicht auf der inneren oder äußeren Wandung des äußeren schlauchförmigen Teiles anzubringen, was in einfacher Weise, beispielsweise durch einen Tauchvorgang, erfolgen kann.

Die Distanzmittel können in verschiedener Weise ausgebildet sein. Eine Ausführungsform besteht darin, daß die Distanzmittel durch Rippen auf der inneren Wandung des äußeren Zuführungs- bzw. Rückführungskanals gebildet sind. Die Rippen lassen sich in sehr einfacher Weise beim Extrudieren des inneren Teiles des Katheters erzeugen. Der äußere, die Außenwandung des äußeren Kanals bildende Teil kann dann anschließend in Form eines Schlauches über den inneren Teil gezogen bzw. geschoben werden. Es ist aber auch denkbar, den gesamten Katheter mit dem äußeren und inneren Teil und den Rippen in einem Stück zu extrudieren.

Im einfachsten Fall verlaufen die Rippen in Längsrichtung des Katheters. Besonders zweckmäßig ist es aber, wenn die Rippen wendelförmig verlaufen. Dadurch ist eine intensive Benetzung der Innenfläche der halbdurchlässigen Membran und damit eine Abführung der von der halbdurchlässigen Membran durchgelassenen Bestandteile sichergestellt. Bei vorgegebenem Fördervolumen der Spülflüssigkeit wird durch die wendelförmige Ausbildung der Rippen außerdem die Strömungsgeschwindigkeit der Spülflüssigkeit erhöht, was weiter eine wirkungsvolle Mitnahme der durch die halbdurchlässige Membran durchgelassenen Bestandteile ermöglicht. Zweckmäßigerweise füllen die Rippen den gesamten radialen Raum des äußeren Kanals aus, so daß sich keine Leckströmung zwischen den äußeren Rändern der Rippen und der benachbarten Wandung ergibt.

Die Zeichnungen zeigen einen Katheter, der nicht Gegenstand der Erfindung ist.
- Fig. 1: zeigt ein erstes Ausführungsbeispiel des Katheters im Bereich seines Endes, und
- Fig. 2: ist ein Schnitt II-II durch Fig. 1,
- Fig. 3: zeigt ein zweites Ausführungsbeispiel eines Katheters, und
- Fig. 4: zeigt einen Schnitt IV-IV durch Fig. 3.

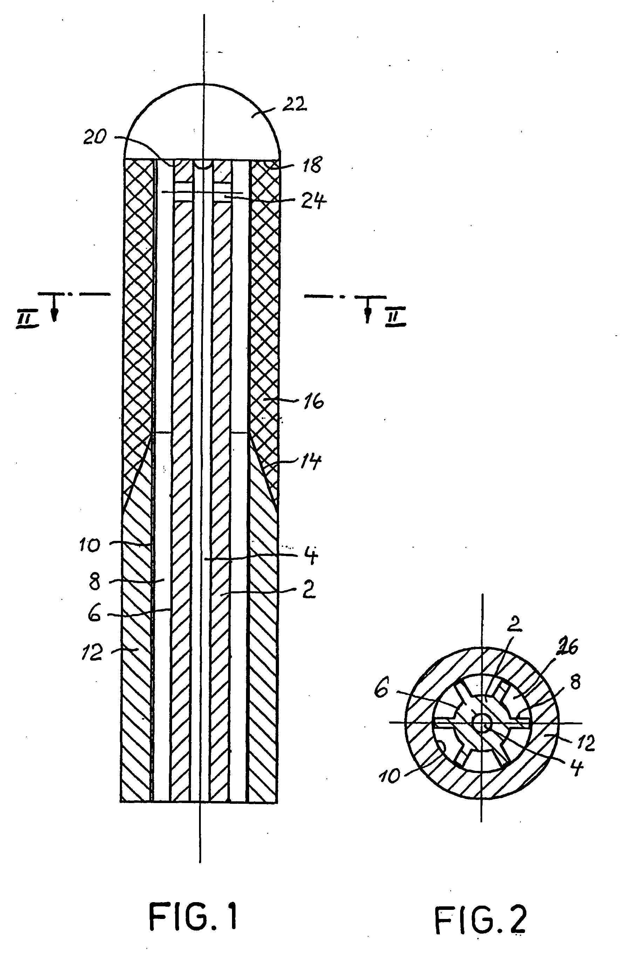

Fig. 1 zeigt ein inneres schlauchförmiges Teil 2, in dem ein Rückführkanal 4 für Spülflüssigkeit verläuft. An einer Außenwandung 6 des schlauchförmigen Teiles 2 befinden sich radial gerichtete Rippen 8, die dicht an eine Innenwandung 10 eines äußeren schlauchförmigen Teiles 12 anschließen. Die Rippen 8 sind durch Extrusion zusammen mit dem inneren schlauchförmigen Teil 2 hergestellt.

Das äußere schlauchförmige Teil 12 geht im Bereich einer konischen Verklebungsfläche 14 in eine halbdurchlässige schlauchförmige Membran 16 über, deren Ende 18 ebenso wie ein Ende 20 des inneren schlauchförmigen Teiles 2 mit einer Kappe 22 verklebt ist, die insgesamt durch einen Klebstofftropfen gebildet sein kann. Der Außendurchmesser des äußeren schlauchförmigen Teiles beträgt bei diesem Ausführungsbeispiel ca. 0,8 mm. Der Rückführkanal 4 ist über radiale Durchbrüche 24 mit einem im Bereich der Rippen 8 gebildeten Zuführkanal 26 verbunden, der in Fig. 2 sichtbar ist, die einen Schnitt II-II durch Fig. 1 zeigt. Der im Bereich der schlauchförmigen Membran 16 verlaufende Teil läßt sich als Sonde bezeichnen.

Bei Gebrauch, beispielsweise zur Messung von Blutzuckerwerten, wird der Katheter wenigstens mit dem Teil der schlauchförmigen Membran 16 mittels einer Kanüle in das Gewebe eingebracht. Danach wird Spülflüssigkeit durch den Zuführkanal 26 zugeführt und durch den inneren Rückführkanal 4 zurückgeführt. Dabei strömt die Spülflüssigkeit an der Innenfläche der schlauchförmigen Membran 16 entlang, wobei die Rippen 8 für in Umfangsrichtung gleichmäßigen Querschnitt des Zuführkanals 26 sorgen und insbesondere auch ein Einfallen der halbdurchlässigen Membran 16 aufgrund des Außendruckes des Gewebes auf die schlauchförmige Membran 16 oder durch einen von dem Rückführkanal 4 erzeugten Unterdruck verhindern. Die an der Innenwandung der schlauchförmigen Membran entlangströmende Flüssigkeit nimmt von der Membran 16 durchgelassene Bestandteile mit, die dann nach Rückführung durch den Rückführkanal 4 gemessen und analysiert werden können.

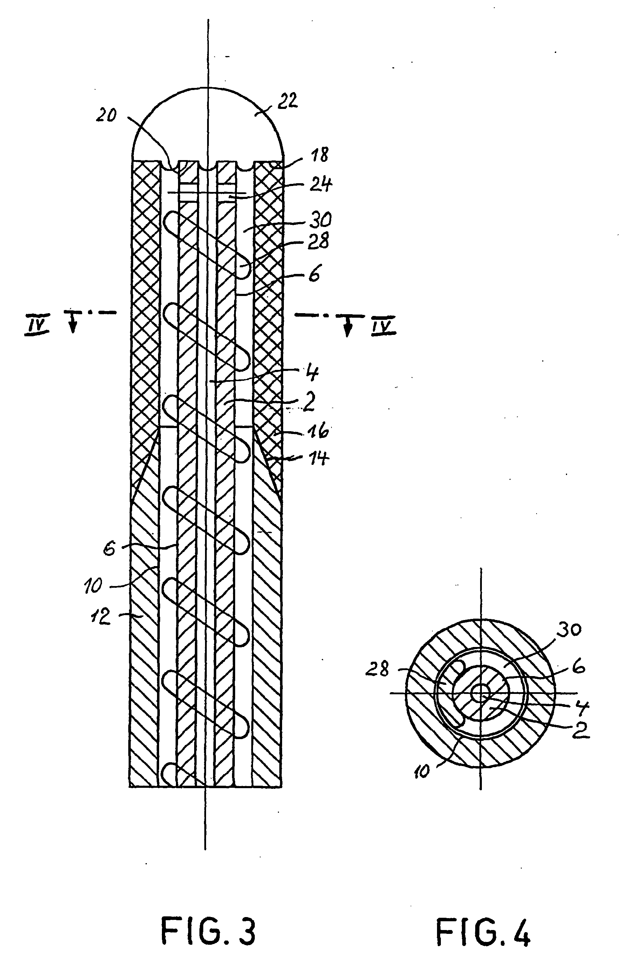

Fig. 3 und 4 zeigen in gleicher Darstellung wie die Fig. 1 und 2 eine Abwandlung des Ausführungsbeisieles gemäß Fig. 1 und 2. Gleiche oder sich entsprechende Teile sind mit gleichen Bezugsziffern versehen. Der Unterschied besteht darin, daß statt der in Längsrichtung verlaufenden Rippen 8 gemäß Fig. 1 eine Wendelrippe 28 vorgesehen ist, die fest auf der Außenwandung 6 des inneren schlauchförmigen Teiles 2 aufliegt oder mit diesem ein Teil bildet. Zwischen den einzelnen Gängen der Wendelrippe 28 ist ein wendelförmiger Zuführkanal 30 für Spülflüssigkeit gebildet und dadurch deren Strömungsweg verlängert und bei vorgegebenem Fördervolumen auch deren Fördergeschwindigkeit im Bereich der schlauchförmigen Membran 16 erhöht, was eine gleichmäßige Benetzung der Innenfläche der schlauchförmigen Membran 16 durch die Spülflüssigkeit und eine intensive Mitnahme der von der schlauchförmigen Membran 16 durchgelassenen Bestandteile bewirkt.

Die Wendelrippe 28 kann in beliebiger Weise ausgebildet werden. Sie kann z. B. aus einem wendelförmigen Draht aus Metall oder Kunststoff bestehen, der auf den inneren schlauchförmigen Teil 2 aufgeschoben wird. Das äußere schlauchförmige Teil 12 und die schlauchförmige Membran 16 werden dann darübergeschoben.

## Patentansprüche

1. Katheter zur Messung chemischer Parameter, insbesondere zum Einführen in biologisches Gewebe, Flüssigkeiten oder dergleichen
mit einem sich in Längsrichtung durch den Katheter bis zu seinem äußeren Ende erstreckenden Zuführkanal (26) zum Zuführen einer Flüssigkeit,
mit einem sich durch den Katheter erstreckenden Rückführkanal zur Rückführung der Flüssigkeit und
mit einer teildurchlässigen Membran (16) am Ende des Katheters, die den Strömungsraum zwischen dem Ende des Zuführkanals (26) und dem Rückführkanal (4) umgibt,
wobei der Zuführkanal (26) den Rückführkanal (4) im wesentlichen koaxial umgibt oder umgekehrt,
wobei die Membran (16) wenigstens im Bereich des Endes des Katheters eine Innenwandung (10) des radial äußeren Rückführkanals bzw. des radial äußeren Zuführkanals (26) bildet,
wobei der Zuführkanal (26) und Rückführkanal (4) am Ende des Katheters durch wenigstens einen radialen Durchbruch (24) miteinander verbunden sind und
wobei der Zuführkanal (26) bzw. der Rückführkanal (4) durch zylindrische Wandungen gebildet ist,
**dadurch gekennzeichnet,**
**daß** die teildurchlässige Membran (16) schlauchförmig ist,
**daß** die zylindrische Wandung des Zuführkanals (26) durch ein schlauchförmiges Teil (12) und die zylindrische Wandung des Rückführkanals durch ein schlauchförmiges Teil (2) gebildet ist,
**daß** Distanzmittel vorgesehen sind, die die Innenwandung (10) des radial äußeren schlauchförmigen Teiles (12) und/oder der schlauchförmigen Membran (16) gegenüber der Außenwandung (6) des inneren schlauchförmigen Teiles (2) abstützen, und
**daß** das äußere schlauchförmige Teil (12) über seine gesamte Länge aus Membranmaterial besteht und bis auf den vorderen Endbereich des Katheters mit einer undurchlässigen Schicht versehen ist.

2. Katheter nach Anspruch 1, **dadurch gekennzeichnet, daß** die Schicht auf der Außen- und/oder Innenseite des äußeren schlauchförmigen Teiles (12) angeordnet ist.

3. Katheter nach Anspruch 1, **dadurch gekennzeichnet, daß** die Distanzmittel durch Rippen (8) auf der Außenwandung (6) des inneren schlauchförmigen Teiles (2) gebildet sind.

4. Katheter nach Anspruch 1, **dadurch gekennzeichnet, daß** die Rippen (8) in Längsrichtung des Katheters verlaufen.

5. Katheter nach Anspruch 1, **dadurch gekennzeichnet, daß** die Rippen (8) wendelförmig als Wendelrippen (28) ausgebildet sind.

6. Katheter nach Anspruch 5, **dadurch gekennzeichnet, daß** die Wendelrippen (28) ein gesondertes Teil bilden, das auf dem radial inneren schlauchförmigen Teil (2) sitzt.

7. Katheter nach einem der Ansprüche 3 bis 6, **dadurch gekennzeichnet, daß** die Rippen (8, 28) in Radialrichtung den äußeren Zuführkanal (30) bzw. Rückführkanal ausfüllen, derart, daß zwischen ihnen ein wendelförmiger Kanal gebildet ist.

## Claims

1. Catheter for measuring chemical parameters, in particular for introduction into biological tissue, fluids or the like, comprising a supply channel (26) extending in the longitudinal direction through the catheter up to its outer end, for supplying a liquid, a return channel extending through the catheter, for returning the liquid and a partially permeable membrane (16) at the end of the catheter, which surrounds the flow chamber between the end of the supply channel (26) and the return channel (4), wherein the supply channel (26) substantially coaxially surrounds the return channel (4) or vice versa, the membrane (16) forming an inner wall (10) of the radially outer return channel or the radially outer supply channel (26), at least in the region of the end of the catheter, wherein the supply channel (26) and return channel (4) are connected to one another at the end of the catheter by at least one radial opening (24) and wherein the supply channel (26) or the return channel (4) are formed by cylindrical walls, **characterised in that** the partially permeable membrane (16) is tubular, **in that** the cylindrical wall of the supply channel (26) is formed by a tubular part (12) and the cylindrical wall of the return channel is formed by a tubular part (2), **in that** spacing means are provided, which support the inner wall (10) of the radially outer tubular part (12) and/or the tubular membrane (16) with respect to the outer wall (6) of the inner tubular part (2), and **in that** the outer tubular part (12), over its entire length, consists of membrane material and is provided with an impermeable layer except for the leading end region of the catheter.

2. Catheter according to claim 1, **characterised in that** the layer is arranged on the outside and/or inside of the outer tubular part (12).

3. Catheter according to claim 1, **characterised in that** the spacing means are formed by ribs (8) on the outer wall (6) of the inner tubular part (2).

4. Catheter according to claim 1, **characterised in that** the ribs (8) extend in the longitudinal direction of the catheter.

5. Catheter according to claim 1, **characterised in that** the ribs (8) are helically configured as helical ribs (28).

6. Catheter according to claim 5, **characterised in that** the helical ribs (28) form a separate part, which is seated on the radially inner tubular part (2).

7. Catheter according to any one of claims 3 to 6, **characterised in that** the ribs (8, 28) in the radial direction fill the outer supply channel (30) or return channel, in such a way that a helical channel is formed between them.

## Revendications

1. Cathéter destiné à mesurer des paramètres chimiques, en particulier destiné à être introduit dans des tissus biologiques, des liquides ou éléments similaires
- comportant un conduit d'admission (26), qui s'étend dans le sens longitudinal à travers le cathéter jusqu'à son extrémité extérieure et qui est destiné à acheminer un liquide,
- comportant un conduit de retour (4), qui s'étend à travers le cathéter et qui est destiné à ramener le liquide et
- comportant une membrane (16) semi-perméable au niveau de l'extrémité du cathéter, laquelle entoure la chambre d'écoulement entre l'extrémité du conduit d'admission (26) et le conduit de retour (4),
- le conduit d'admission (26) entourant sensiblement coaxialement le conduit de retour (4) ou inversement,
- la membrane (16) formant au moins dans la zone de l'extrémité du cathéter une paroi intérieure (10) du conduit de retour (4) radialement extérieur ou du conduit d'admission (26) radialement extérieur,
- le conduit d'admission (26) et le conduit de retour (4) communiquant l'un avec l'autre au niveau de l'extrémité du cathéter par au moins un trou débouchant (24) radial, et
- le conduit d'admission (26) ou le conduit de retour (4) étant formés par des parois cylindriques,
**caractérisé**
- **en ce que** la membrane (16) semi-perméable est conçue sous forme tubulaire,
- **en ce que** la paroi cylindrique du conduit d'admission (26) est formée par une partie tubulaire (12) et la paroi cylindrique du conduit de retour (4) est formée par une partie tubulaire (2),
- **en ce qu'**il est prévu des moyens d'écartement, qui supportent la paroi intérieure (10) de la partie tubulaire (12) radialement extérieure et/ou la membrane tubulaire (16) par rapport à la paroi extérieure (6) de la partie tubulaire intérieure (2), et
- **en ce que** la partie tubulaire extérieure (12) est réalisée sur toute sa longueur dans un matériau pour membrane et est revêtue d'une couche imperméable à l'exception de la zone d'extrémité antérieure du cathéter.

2. Cathéter selon la revendication 1, **caractérisé en ce que** la couche est déposée sur la face extérieure et/ou la face intérieure de la partie tubulaire extérieure (12).

3. Cathéter selon la revendication 1, **caractérisé en ce que** les moyens d'écartement sont formés par des nervures (8) sur la paroi extérieure (6) de la partie tubulaire intérieure (2).

4. Cathéter selon la revendication 1, **caractérisé en ce que** les nervures (8) s'étendent dans le sens longitudinal du cathéter.

5. Cathéter selon la revendication 1, **caractérisé en ce que** les nervures (8) sont conçues sous forme de nervures hélicoïdales (28).

6. Cathéter selon la revendication 5, **caractérisé en ce que** les nervures hélicoïdales (28) forment une partie séparée qui est emmanchée sur la partie tubulaire (2) radialement intérieure.

7. Cathéter selon l'une quelconque des revendications 3 à 6, **caractérisé en ce que** les nervures (8, 28) remplissent dans le sens radial le conduit d'admission (30) extérieur ou le conduit de retour, de telle sorte qu'il se forme un conduit hélicoïdal entre lesdites nervures.
